# EUROPEAN PATENT SPECIFICATION

(11) **EP 3 376 974 B1**
(45) Date of publication and mention of the grant of the patent: **02.04.2025**
(21) Application number: 16810098.0
(22) Date of filing: 18.11.2016
(51) Int. Cl.: A61B 17/29, A61B 34/00, A61B 17/00, A61B 90/00

(54) **SURGICAL INSTRUMENT**
CHIRURGISCHES INSTRUMENT
INSTRUMENT CHIRURGICAL

(30) Priority: 20.11.2015 NL 2015829
(43) Date of publication of application: 26.09.2018
(73) Proprietor: Endoscopic Forcereflecting Instruments B.V., 7521 PH Enschede (NL)
(72) Inventor: VLEUGELS, Michel Petronella Hubertus, 6581 PX Malden (NL)
(74) Representative: EP&C
(86) International application number: PCT/NL2016/050809
(87) International publication number: WO 2017/086792

(56) References cited:
- EP-A1- 2 881 067
- EP-A2- 1 423 056
- WO-A1-2010/138083
- CN-A- 104 095 669
- JP-A- 2014 108 344
- US-A1- 2012 116 364
- US-A1- 2012 116 391
- US-A1- 2013 324 999
- US-A1- 2014 025 067

## Description

The present invention relates to a surgical instrument according to the preamble of independent claim 1. Surgical instruments of this type are already known, for example from European Patent EP1423056 B1.

Such surgical instruments become increasingly popular as they can be used during minimally invasive surgery. Minimally invasive surgery is usually carried out by making multiple small incisions in the body of the patient, through which the remote surgical instruments, such as cameras, scissors, staplers and forceps can be inserted. To allow for insertion through small incisions, the instruments that are used for minimally invasive surgery generally comprise elongated thin sections, wherein a working element is arranged on one end thereof and a hand piece is arranged on another end thereof.

EP1423056 B1 discloses a surgical instrument for the use in minimally invasive surgery, wherein a sensor is arranged in a working element, providing feedback on the force that is applied by the working element to the target tissue. The instrument comprises a proximal part and a distal part, which are releasably connected. The use of an optical sensor comprising a glass fibre was disclosed. The glass fibre was guided from the distal part, through an elongated shaft, towards the proximal part and a control unit.

In the surgical instrument of EP1423056 B1 an optical sensor is arranged in the distal part of the instrument. Therefore, an optical connection needs to be provided for the transmission of the optical signal between the glass fibre in the proximal part and the glass fibre in the distal part. As a result, the distal part is relatively complex with the presence of the optical sensor and the optical connector in the distal part and thereby less suitable for single use and disposal afterwards.

Further, the surgical instrument of EP1423056 B1 comprises an optical connector. Optical connectors are known, for example from US 6,574,401 B2, to deteriorate after repeated sterilization operations because of the formation of micro-cracks in the interface surfaces. The quality of the provided feedback signal from the disclosed surgical instrument is therefore expected to decrease over time, giving rise to a less accurate instrument.
From US 2013/324999 A1, a surgical instrument is known, which comprises a body and end effector and which further comprises an energy component to activate the end effector. The instrument further comprises an orientation sensor and a control module, which is configured to set an energy setting for the energy component in response to a sensor signal from the orientation sensor.
In JP 2014/108344 A, a surgical instrument is disclosed, which comprises a pressure-sensitive sensor and which is configured to issue an alarm when a reference force value is exceeded.
In CN 104095669 A, a surgical operating clamp is disclosed, which comprises a piezoelectric force sensor and which is configured effect an alarm when a clamping force exceeds a preset threshold value.
US 2014/025067 A1 discloses a surgical instrument with an end effector, having two jaw members, further comprising optical fibers, which are disposed in the jaw members.
In WO 2010/138083 A1, a robotic manipulator is disclosed for use in flexible enndoscopy.
In EP 2 881 067 A1, a surgical instrument is disclosed, which comprises a motor drive unit and a measuring arrangement, which is arranged on a drive train of the instrument and which is configured to measure a load in the drive train.

US 2012/116364 A1 and US 2012/116391 A1 disclose further surgical instruments with a working element arranged on a distal end of an elongated shaft that measure a force applied by a trigger to actuate the working element.

It is an object of the present invention to provide a surgical instrument that lacks at least one of the above-mentioned drawbacks of the known surgical instrument or at least to provide an alternative surgical instrument.

The present invention provides a surgical instrument as claimed in claim 1. Further embodiments are disclosed by the dependent claims 2- 14. The disclosure further provides a method to determine a force exerted on an at least one working element of a surgical instrument.

The surgical instrument comprises a distal part, which serves to be at least partially inserted in the patient's body during the surgery. The distal part comprises an elongated shaft, which preferably is a hollow elongated shaft to provide a channel there through, extending from a first end of the elongated shaft to a second end of the elongated shaft. The distal part further comprises at least one working element which is movably mounted on a distal end of the elongated shaft.

In an embodiment, the at least one working element is rotatably mounted on the elongated shaft and is configured to be rotated with respect to the elongated shaft. An example of a suitable working element is a forceps which is configured to be rotatably mounted on a distal end of the elongated shaft.

The at least one working element serves to execute certain operations in the interior of the patient's body, while the actuation for this operation is performed outside the patient's body. The benefit of having the actuation outside the patient's body is the fact that certain elements, for example electric components such as driving motors, can be situated outside the patient's body. This will increase the working space of the surgeon in the patient's body or will reduce the amount of damaged tissue, resulting from the incision.

To enable movement of the working element, the surgical instrument comprises an actuation rod. A first end of the actuation rod is connected to the at least one working element. The actuation rod extends, preferably substantially parallel to the elongated shaft, from a distal end of the elongated shaft to a proximal end of the elongated shaft.

The actuation rod is configured to transfer a movement that is applied to a second end of the actuation rod to the at least one working element or vice versa. In an embodiment, a translational movement of the actuator device is converted into a rotational movement of the at least one working element.

In an embodiment, the actuation rod is arranged in the interior of the hollow elongated shaft. When the actuation rod is situated in the interior of the hollow elongated shaft, the perimeter of actuation rod will not come into contact with the surrounding tissue of the patient's body. An advantage of this is that no additional friction will occur when the actuation rod is moved with respect to the stationary hollow elongated shaft and that, as a result of fewer cavities and slits, the risk for contamination of the instrument is reduced. Another advantage of arranging the actuator rod at least partially in the interior of the hollow elongated shaft is that the surrounding tissue is less likely to block moving elements, like the actuation rod, in the surgical instrument.

The surgical instrument comprises a proximal part, wherein the proximal part is releasably connected to the distal part. The connection between the proximal part and the distal part is made releasable to allow for separate handling of the proximal part and the distal part of the instrument, after use in surgery.

When the proximal and the distal part of the surgical instrument are releasable from one another, both parts can for example be sterilized separately. Certain elements in the instrument, for example electronics, cannot withstand the consequences of sterilization with gamma ray radiation and/or very high temperatures. Therefore, the distal part can be sterilized separately from the proximal part in order to allow for optimum sterility of both parts.

When the distal part can be separated from the proximal part, the distal part can be provided as a disposable part as well. Since the non-mechanical components, such as sensors and electrical components, are arranged in the proximal part, the distal part contains only mechanical parts. These mechanical parts are relatively in-expensive and may, in order to achieve optimal sterility, be disposed after a single usage.

A further advantage of having a distal part, free of electronic components, is that these electronic components cannot influence any observations that are made in the body. For example, when the instrument is applied for surgery, together with in-situ MRI observations, the lack of electronic components in the distal part of the instrument will give rise to less interference between the MRI field and electro-magnetic fields that are generated by electronic components.

Additional, the distal part may, in an embodiment of the instrument, be sensor-free. With sensor-free, it is meant that no electronic or optical sensor elements are arranged in the distal part. This not only provides the advantage of having less influence of the instrument on measurements in the patient's body, but further provides for lower costs of the distal part.

Another advantage of the distal part to be releasable from the proximal part is that multiple types of distal parts can be connected to a single type of proximal parts. Since the proximal part contains expensive components, such as sensors and electrical components, the proximal part is designed to be universally applicable. Whereas a multitude of different distal parts, having different kinds of working elements, can be connected to the proximal part. However, in alternative embodiments, multiple types of proximal parts may be provided, such as hand-held proximal parts of different sizes or shapes.

In an embodiment, the distal part may be rotatable with respect to the proximal part around the elongate direction of the elongated shaft and the actuation. During rotation, the shaft and the actuation rod, which is preferably arranged concentrically with the rod, remain stationary with respect to each other and the working element will remain stationary with respect to the elongated shaft.

When the proximal part is kept stationary and the distal part is rotated, the orientation of the working element can be changed with respect to the proximal part. In case, for example, tissue needs to be grasped under a different angle, the distal part may be rotated in order to change the orientation of the working element without the need to substantially change a position and/or orientation of the hand of the surgeon that operates the instrument. A rim or other gripping element may be arranged on the distal part, in order to be gripped by a surgeon for rotating the distal part with respect to the proximal part.

To facilitate the rotation, the coupling between the actuation rod and the connection element is rotatable as well, thereby allowing a similar force with similar force-feedback to be transmitted by the connection element, independent of its position.

Furthermore, when no electronic and/or optical sensor elements are arranged in the distal part, rotation of the distal part with respect to the proximal part is not hindered by signal connections to be made between the distal part and the proximal part. In such embodiment, the angle of rotation of the distal part with respect to the proximal part may be endless.

The proximal part comprises an actuator device, the actuator device is releasably connected to the second end of the actuation rod. The connection between the actuator device and the second end of the actuation rod is made releasable as well, to allow for the separation of the distal part, comprising the actuation rod, from the proximal part, comprising the actuator device for above-mentioned reasons.

The actuator device is configured to move the at least one working element with respect to the elongated shaft. In order for the at least one working element to move, the actuator device transfers a movement to the actuation rod after which the actuation rod transfers this movement to the at least one working element, or vice versa. In an embodiment according to the invention, a translational movement of the actuator device is transferred to a translational movement of the actuation rod, which is in turn transferred to a rotational movement of the at least one working element.

The surgical instrument comprises a sensor, which is arranged to transmit a signal that is representative for a force exerted on the at least one working element. The measurement of the force on the at least one working element is useful in order to provide feedback about the exerted force on the at least one working element to the operator of the instrument. When a forceps is arranged as a working element for example, the clamping force of the forceps can be measured with the sensor and the operator can determine whether either additional clamping, with increased clamping force, or loosening of the forceps is needed.

In an embodiment, the surgical instrument is a surgical instrument that is used for minimally invasive surgery, whereby the surgery is performed through several small incisions and less damage is done to the patient's body during surgery.

The sensor is arranged in the actuator device, to measure force and/or strain exerted on the actuator device. Since the force and/or strain exerted on the actuator device is representative for a force exerted on the at least one working element, the signal provided by this force and/or strain sensor can be used.

Since the sensor is arranged in the proximal portion of the instrument, no releasable sensor signal connection between the proximal part and the distal part of the instrument has to be provided in order for the transmission of the signal measured with the sensor.

The distal part can be provided as a disposable part. When the distal part is disposable, no further sterilization of the distal part is required after the use in surgery, thereby reducing the effort that needs to be made in order to keep the instrument operable.

The magnitude of the signal that is measured by the sensor in the actuator device is generally not the same as it would be on the at least one working element, because of a transmission between the movement of the at least one working element and the movement of the actuator device. Therefore, the ratio between a force on the at least one working element and the resulting force on the actuator device needs to be known, in order to provide accurate feedback to the operator.

The sensor in the actuator device is configured to measure a force and/or strain, whereby this force and/or strain can be applied by the operator with the actuator device, and/or it can be a resulting force from a force that is applied back to the instrument by the tissue that is grasped.

An example of such a case is the grasping of a blood vessel, which expands and contracts, whereby a force is applied to the surgical instrument, during a pulse from the beating heart. As a result of this, the operator would be able to accurately determine the heartbeat of the patient, but would in general also be able to judge whether the tissue, that is grasped, comprises an artery. In another example, the response of the grasped tissue to a clamping force, e.g. the impedance of the tissue, can be measured with the instrument. When a layer of tissue is firm, for example when a layer of muscles is grasped, the response will be stronger than when a softer tissue layer was grasped.

The actuator device comprises a connection element connected to the second end of the actuation rod, wherein the sensor is arranged on the connection element. The connection element is arranged in a distal portion of the actuator device, through which the movement of the actuator device is transferred to the actuation rod. As a result of the arrangement with the sensor on the connection element, a relatively low amount of friction in for example the remainder of the actuator device, can disturb the measurement of the force that is exerted on the at least one working element.

In an embodiment, the connection element is arranged such, that the force and/or strain that is transmitted through the connection element is substantially uniaxial. The advantage of having a uniaxial force and/or strain is that the sensor only needs to be configured to measure force and/or strain in a direction substantially parallel to the direction of the largest component of the force and/or strain in the connection element.

In an embodiment, the connection element comprises a thinned section in which the cross sectional area of the connection element is substantially smaller than the cross sectional area in the remainder of the connection element. The strain in the thinned section, resulting from the applied force and/or strain to the connection element will be substantially larger as compared to the strain in a thicker part of the connection element. When the sensor is arranged in the thinned section, the strain can be measured more accurately since the absolute value of the strain is higher, when compared to a case wherein the sensor was placed in the thicker part of the connection element.

In an embodiment, the connection element, and in particular the thinned section thereof, may align itself with a direction in which the force is applied on the connection element. In order to align itself, the connection element may be rotatably mounted to the actuation rod.

As a result of this aligning, the force through the connection element may become parallel to the direction in which the sensor is configured to measure, in order to optimize the measured signal from the sensor.

The connection element of the actuator device and the second end of the actuation rod are releasably connected. The connection between the connection element and the second end of the actuation rod is configured to be releasable, in order to allow for the separation of the distal part, comprising the actuation rod, from the proximal part, comprising the actuator device. The connection between the connection element and the second end of the actuation rod can preferably be a snap fastener in which the second end of the actuation rod comprises a male insertion piece to be inserted in, and releasably connected to, a female cavity in the connection element of the actuator device.

In an embodiment, the sensor is an optical sensor, in particular a Fibre Bragg Grating, wherein the sensor is configured to measure mechanical strain in the connection element of the actuator device. Fibre Bragg Grating sensors rely on the reflection of a specific wavelength spectrum of an incident light beam through a glass fibre. In the grating, a wavelength-specific dielectric mirror is arranged, through which a portion of the incident light beam, having a specific corresponding wavelength spectrum, is reflected.

When the glass fibre with the grating is arranged on the connection element in the surgical instrument, the grating will be strained along with the connection element when a force and/or strain is applied to the connection element. As a result of this, the distance between the dielectric mirror elements in the grating will vary, which also causes the wavelength spectrum of the reflected portion of the incident light beam to change.

When the reflected portion of the light beam is analysed in a spectrometer, the amount of strain in the grating, and therefore the amount of strain in the connection element can be determined. The advantage of using a Fibre Bragg Grating sensor over other sensor types, such as for example strain gauges, is that a Fibre Bragg Sensor contains no electrical components, since the working principle of the sensor is based on the transmission and reflection of light. Instead of having an electrical signal, which may influence or be influenced by other surgical equipment, such as lamps and/or imaging devices, a glass fibre, or the like, is provided through which an optical signal is guided.

A drawback of the use of a Fibre Bragg Grating is its temperature dependence. If the temperature of the grating changes, the strain resulting from the thermal expansion and/or thermal contraction of the grating will cause the reflected wavelength spectrum to vary as well. To compensate in an embodiment for this temperature-induced strain of the signal, a second optical sensor, in particular a second Fibre Bragg Grating is arranged in the proximal part. The second sensor will be arranged in a portion of the proximal part that is not exposed to a force and/or strain that results from mechanical loading. Generally, the second sensor is arranged in the same glass fibre as the first sensor. However, the second sensor will be arranged in a part of the glass fibre that is not prone to mechanical loading. Therefore, the second sensor will be solely prone to strain resulting from the thermal expansion and/or the thermal contraction of the grating. Therefore, when the reflected wavelength pattern of the second sensor is subtracted from the wavelength pattern of the first sensor, the remaining shift in reflected wavelength will be solely caused by the force and/or strain in the connection element, resulting from the mechanical loading of the connection element.

In an embodiment according to the invention, the mechanical strain in the connection element of the actuator device is representative for the force exerted on the at least one working element. When a force is applied to the at least one working element, the force is transmitted to the connection element through the actuation rod and vice versa. This transmission induces a certain ratio between the force that is applied to the at least one working element and the force that is applied to the connection element. This ratio can be dependent on the position of the at least one working element with respect to the elongated shaft.

Another factor that induces a difference in ratio between the force that is applied to the at least one working element and the force in the connection element is the friction within the instrument between the sensor and the at least one working element. All movable elements in the surgical instrument will generate a frictional force, which counteracts the movement that is induced by the actuation of the actuator device. To provide an accurate feedback signal to the operator of the instrument, the signal from the sensor in the connection element is therefore corrected for the frictional losses in the instrument.

In an embodiment according to the invention, the surgical instrument comprises a processing unit, wherein the processing unit is configured to determine a force exerted on the at least one working element on the basis of the signal of the sensor. The processing unit is therefore configured to change the signal that was measured with the sensor on the connection element, in order to allow for the correction of the influence of temperature, friction and a transmission ratio between the at least one working element and the connection element.

Therefore, the processing unit comprises the earlier mentioned spectrometer to analyse the reflected wavelength patterns from the Fibre Bragg Grating on the connection element and the second Fibre Bragg Grating in the proximal part. The processing unit further comprises a calculating device to correct the measured mechanical strain in the connection element for the transmission ratio in order to provide a feedback signal on the applied force exerted on the at least one working element to the user.

In an embodiment according to the invention, a position sensor is arranged in the proximal part of the surgical instrument. The position sensor is configured to transmit a signal that is representative for the position of the at least one working element with respect to the elongated shaft of the distal part. The mentioned friction of the various elements in the instrument, stretching between the at least one working element and the connection element of the actuator device can be dependent on the position in which the at least one working element, and thereby the actuation rod and the connection element as well, is with respect the stationary elongated shaft of the instrument. When a position sensor is arranged in the instrument, the position of the movable elements can be determined after which the signal is transmitted to the processing unit. When the surgical instrument is carefully calibrated, using a calibration method that is not part of the invention, the processing unit is configured to determine the friction in the instrument, given the measured position of the at least one working element, and is configured to correct the feedback signal for the friction in the instrument.

In an embodiment, the surgical instrument comprises a housing, wherein the actuator device is arranged at least partially in the interior of the housing and wherein the elongated shaft is releasably connected to the housing, resulting in a substantially rigid connection.

The position sensor is arranged in the housing, wherein a first end of the sensor is connected to the housing and a second end of the sensor is connected to the actuator device and wherein the position sensor is configured to measure the position of the actuator device with respect to the housing. The position of the at least one working element with respect to the elongated shaft is dependent on the position of the actuator device with respect to the housing, since the at least one working element is both connected with the actuator device, through the actuation rod and the connection element, and connected with the housing through the elongated shaft.

The processing unit is configured to change the signal of the position sensor into a signal that is representative for the position of the working element. When the position of the at least one working element is known, the processing unit is configured to relate the force exerted on the at least one working element to the position of the at least one working element with respect to the elongated shaft of the distal part, in order to provide information to the operator, about the force applied with the actuator device.

According to the invention, the actuator device comprises a trigger, wherein the trigger is configured to transfer a movement to the connection element of the actuator device. The trigger is arranged at least partially in the interior of the housing and will extend outward from the interior of the housing to be accessible for the operator. The force that is applied to the trigger will be mechanically transmitted through the connection element and the actuation rod towards the at least one working element. In turn, a restrictive force from the at least one working element can be sensed in the trigger as well.

In another embodiment, the actuator device comprises an actuator, for example an electric motor. The actuator is arranged at least partially in the interior of the housing of the instrument, wherein the actuator is configured to move the connection element of the actuator device, in particular with respect to the stationary housing. Thereby, the actuator will also move the at least one working element with respect to the elongated shaft, via the actuation rod.

In another embodiment, both a trigger and an actuator are arranged in the proximal part, wherein both the trigger and the actuator are connected to the actuator device in series. This means that a movement of the trigger with respect to the housing of the proximal part will result in a movement of the actuator and vice versa. The connection element, actuation rod and the at least one working element are finally moved by the combined movement of the actuator and the trigger. An advantage of the in series connection of the trigger and the actuator is that when either one of them is in a certain position or applies a certain force to the connection element, the other can be moved or can be exposed to a force, after which the connection element, and the connected actuation rod and the at least one working element, will move to another position.

In an embodiment, the processing unit is configured to change the movement of the actuator. The actuator can for example be an electrical stepper motor or a solenoid, which is configured to move when a potential is applied to the actuator. The amount of movement or force can therefore be controlled by changing the applied potential. This means that, in an embodiment, the applied force or movement of the actuator can be changed, dependent on the measured value for the force exerted on the at least one working element, wherein the processing unit is configured to construct a feedback loop in which the force exerted on the at least one working element is measured and evaluated, after which the amount of force that is applied by the actuator is either altered or not.

Another advantage of an arrangement wherein the processing unit provides feedback to the actuator is that, when a force is, for example by accident, applied by the operator on the trigger which could harm the tissue that is to be treated. In such a case, the processing unit can provide a signal to the actuator to apply a negative force to the connection element in order to reduce the net force that is applied to the tissue with the at least one working element.

In an embodiment of the surgical instrument, the at least one working element is a forceps. A forceps is known from the state-of-the-art and has been used often during minimally invasive surgery. In a forceps two working arms are arranged, which are rotatably mounted on the elongated shaft and can be rotated with respect to the elongated shaft and with respect to each other. When a force needs to be applied to the forceps, the actuator device will move the connection element towards the proximal direction, after which the actuation rod is pulled in a direction away from the distal end of the elongated shaft, after which the grasping ends of the forceps are rotated to one another.

In another embodiment, the at least one working element can be part of a stapler for the closing and connecting of two adjacent layer of tissue. Prior to the stapling of these layers, it is advantageous to determine the thickness and the rigidity of the tissue layers. A surgical instrument, may be configured to measure the position of the at least one working element and to measure the force that is exerted on the at least one working element. With these parameters, the processing unit of the instrument according to the disclosure is configured to determine the thickness and rigidity of the tissue layers.

Further characteristics and advantages of the surgical instrument according to the invention as defined by the claims will be explained in more detail below with reference to an embodiment which is illustrated in the appended drawings, in which:
Figure 1 schematically depicts an embodiment of a surgical instrument according to the invention;
Figure 2 depicts the embodiment of Figure 1 in which the distal part and the proximal part are spaced from each other; and
Figure 3 schematically depicts an embodiment of the actuator device of the surgical instrument.

Throughout the figures, the same reference numerals are used to refer to corresponding components or components which have a corresponding action.

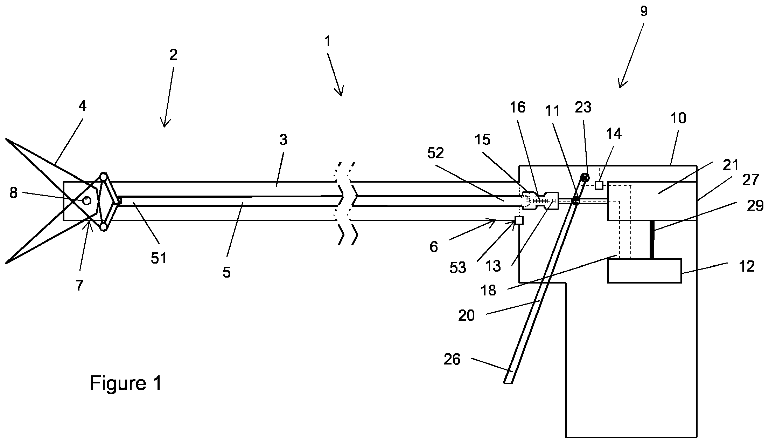

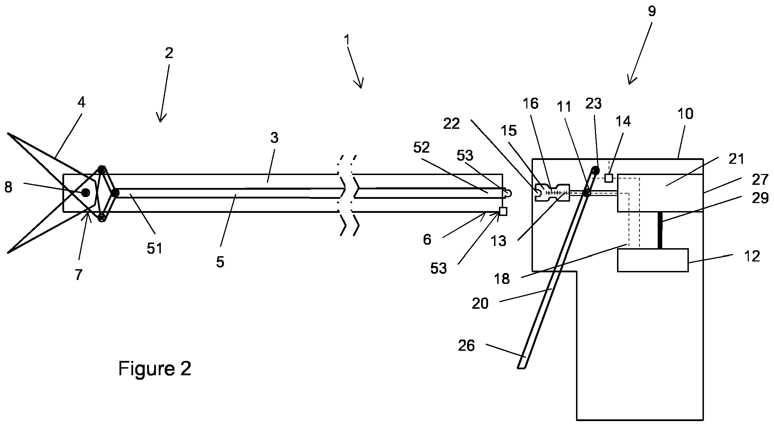

Figures 1 and 2 show an embodiment of the surgical instrument according to the invention, denoted by referral number 1. The surgical instrument 1 comprises a proximal part 9 and a distal part 2, wherein the distal part 2 comprises a hollow elongated shaft 3, a forceps 4 and an actuation rod 5. In Figures 1 and 2, the hollow elongated shaft 3 is displayed in two pieces, separated by a zigzag cut, to highlight the large, but undefined length of the hollow elongated shaft 3.

The surgical instrument in Figure 1 is displayed in a connected operative configuration, wherein the distal part 2 of the surgical instrument 1 is connected to the proximal part 9 thereof.

The surgical instrument in Figure 2 is displayed in a separated handling configuration, wherein the distal part 2 is not connected to the proximal part 9, in order to allow for separate handling of the distal part 2 and the proximal part 9.

The hollow elongated shaft 3 extends from a proximal end 6 thereof to a distal end 7 thereof, wherein the actuation rod 5 is arranged at least partially in the interior of the hollow elongated shaft 3.

The forceps 4 is movably mounted on the hollow elongate shaft 3, wherein the forceps 4 is configured to rotate with respect to the hollow elongate shaft 3 round hinge point 8. A first end 51 of the actuation rod 5 is rotatably connected to the forceps 4 such, that a translational movement of the actuation rod 5 substantially parallel to the length of the actuation rod 5 is translated in a rotational movement of the forceps 4 round hinge point 8.

In the embodiment, the distal part 2 is sensor-free, such that no electronic or optical sensor elements are arranged in the distal part 2. The distal part 2 thereby comprises mechanical components for transmitting forces between the proximal part 9 and the forceps 4.The proximal part 9 of surgical instrument 1 comprises a housing 10, an actuator device 11, a processing unit 12, a temperature sensor 13 and a position sensor 14.

The housing 10 is releasably connected to the proximal end 6 of the hollow elongated shaft 3, wherein a second end 52 of the actuation rod 5 protrudes partially into the interior of the housing 10 of the proximal part 9. On the proximal end 6 of the hollow elongated shaft 3, a coupling element 53 is arranged, which is configured to releasably connect the hollow elongated shaft 3 to the housing 10.

The actuator device 11 is arranged at least partially in the interior of the housing 10, wherein the second end 52 of the actuation rod 5 is releasably connected to a connection element 15 of the actuator device 11 and wherein the actuator device 5 is configured to move the forceps 4 with respect to the hollow elongated shaft 3 through actuation rod 5.

A sensor is arranged on the connection element 15, wherein the sensor is a Fibre Bragg Grating 16 and wherein the Fibre Bragg Grating 16 is configured to transmit a signal that is representative for the force exerted on the forceps 4. In order to transmit a signal that is representative for the force exerted on the forceps 4, the mechanical strain in the connection element 15 is measured with the Fibre Bragg Grating 16. The mechanical strain and/or a force in the connection element 15 is representative for the force exerted on the forceps 4 since the forceps 4 and actuation rod 5 have a certain transmission ratio.

The processing unit 12 is arranged in the interior of the housing 10, wherein the processing unit 12 is configured to determine a force exerted on the forceps 4 on the basis of the signal from the Fibre Bragg Grating 16. A first glass fibre 17 is arranged in the interior of the housing 10, wherein the first glass fibre 17 is configured to transmit an optical signal from the Fibre Bragg Grating 16 to the processing unit 12 and vice versa.

The position sensor 14 is arranged in the interior of the housing 10, wherein the position sensor 14 is configured to transmit a signal that is representative for the position of the forceps 4 with respect to the hollow elongated shaft 3. In order to transmit a signal that is representative for the position of the forceps 4, the position sensor 14 is configured to measure the position of the actuator device 11 with respect to the housing 10. A second glass fibre 18 is arranged in the interior of the housing 10, wherein the second glass fibre 18 is configured to transmit a signal from the position sensor 14 to the processing unit 12 and vice versa. The processing unit 12 is configured to relate the force exerted on the forceps 4 to the position of the forceps 4 with respect to the hollow elongated shaft 3.

The temperature sensor 13 is arranged in the interior of the housing 10, wherein the temperature sensor 13 is configured to transmit a signal that is representative for the temperature in the housing 10. Since the principle of the Fibre Bragg Grating 16 is based on the measurement strain by reflected wavelength patterns on a dielectric mirror, a strain resulting from the thermal expansion of the housing 10, following a change in ambient temperature, will cause a disturbance in the measured signal. Therefore, a second Fibre Bragg Grating will be arranged a temperature sensor 13, wherein the second Fibre Bragg Grating is configured to transmit a signal that is representative for the temperature in the first glass fibre 17. The first glass fibre 17 is configured as well to transmit a signal from the temperature sensor 13 to the processing unit 12 and vice versa. The processing unit 12 is configured to correct the signal from the first Fibre Bragg Grating 16 with the measured signal for the thermal expansion of the housing 10.

The distal part 2 and the proximal part 9 of the surgical instrument 1 are releasably connected. When the distal part 2 and the proximal part 9 are released from one another, the parts can be sterilized separately. The distal part 2 for example, comprises no electrical components and can for example, be sterilized by means of gamma ray radiation. Due to the absence of expensive electrical components in the distal part 2, the distal part 2 can as well be made disposable. The proximal part 9, however, contains electrical components, such as the processing unit 12. Since electrical components can generally suffer from exposure to gamma ray radiation, the proximal part 9 can be sterilized for example with steam.

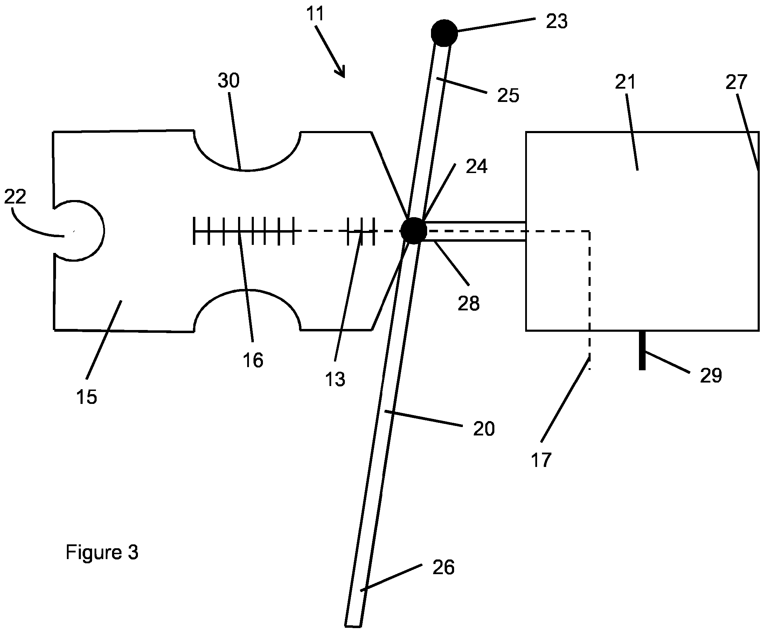

Figure 3 shows an embodiment of the actuator device 11 of the surgical instrument 1 according to the invention. The actuator device 11 comprises a connection element 15, a Fibre Bragg Grating 16, a temperature sensor 13, a glass fibre 17, a trigger 20 and actuator 21. The connection element 15 is configured to receive male insertion piece 53 of a second end 52 of an actuation rod 5 in the female cavity 22.

In the embodiment, the male insertion piece 53 is rotatable within the female cavity 22, such that the connection element 15 will align itself in a direction which is similar to the direction in which a force is applied on the connection element 15 in order to obtain a substantially uniaxial stress in the connection piece 15.

Furthermore, both the male insertion piece 53 and the female cavity 22 have circular cross section, perpendicular to the elongate direction of the rod 5. These circular cross sections provide that the male insertion piece 53 of the rod 5 can be rotated within the female cavity 22 during rotation of the distal part 2 with respect to the proximal part 9.

The connection element 15 comprises a thinned section 30, where the cross sectional area of the connection element 15 is substantially smaller than the cross sectional area in the remainder of the connection element 15. A result of this is that the strain, as a result of the force exerted on the forceps 4, is larger in the thinned section 30 as compared to the strain in the remainder of the connection element 15.

In the embodiment, the transition in cross section between the thinned section 30 of the connection element 15 and the remainder of the connection element 15 is made gradually. By preventing the presence of sharp edges in the connection element 15, fewer stress concentrations will occur, so that the stresses in the thinned section 30 are fully dependent, preferably linearly dependent, on the force that is applied at the forceps 4.

A Fibre Bragg Grating 16 is arranged on the thinned section 30 of the connection element 15, wherein the Fibre Bragg Grating 16 is configured to transmit a signal that is representative for the force exerted on forceps 4, whereto the mechanical strain in the connection element 15 is measured with the Fibre Bragg Grating 16. The strain is measured in the thinned section 30, since the higher strain in the thinned section 30, as compared to the strain in the remainder of the connection element 30, can be detected by the Fibre Bragg Grating 16 more accurately.

The temperature sensor 13 is arranged in the first glass fibre 17 on a portion of the actuator device 11 that is prone to substantially zero loading, especially when compared to the force present in the thinned section 30 of the connection element 15. Therefore, the temperature sensor 13 is configured to only measure strain resulting from the thermal expansion of the first glass fibre 17 and no strain, resulting from the mechanical loading of the actuator device 11.

The trigger 20 of the actuator device 11 is arranged partly in the interior of the housing 10 of the surgical instrument 1, wherein the trigger 20 is configured to transfer a movement to the connection element 15 of the actuator device 11. A distal portion 25 of the trigger 20 is rotatably mounted on the housing 10 and is configured to rotate with respect to the housing 10 round hinge point 23. A proximal portion 26 of the trigger 10 extends outward from the housing 10, wherein the proximal portion 26 is configured to be moved by the operator of the surgical instrument 1. As a result of this movement, the trigger 20 will rotate round hinge point 23. The trigger 20 is rotatably mounted to the connection portion 15 in hinge point 24 as well. As a result of a rotation of the trigger 20 round hinge point 23, the connection element 15 is forced to move in a direction substantially parallel with the actuation rod 5. A movement of the trigger 20 therefore results in a movement of the forceps 4, through connection element 15 and actuation rod 5.

The actuator 21 of the actuator device 11 is arranged in the interior of the housing 10, wherein the actuator 21 is configured to move the connection element 15 of the actuator device 11. A first end 27 of the actuator 21 is mounted on the housing 10 of the instrument 1 and a second end 28 of the actuator 21 is rotatably connected to the connection element 15 in hinge point 24.

The actuator 21 is configured to apply a force to the connection element 15, dependent on the force exerted on the forceps 4. Thereto, the actuator 21 is connected to the processing unit 12 through cable 29, wherein the cable 29 is configured to transport an electric steering signal from the processing unit 12 to the actuator 21 and vice versa. When the force, as applied by the operator through the trigger 21, exerted to the forceps 4 is too low, the processing unit 12 is configured to send a signal to the actuator 21 through cable 29. The signal dictates the actuator 21 to apply a force to the connection element 15, whereby the combined force of the trigger 20 and actuator 21 on the connection element 15, and thus on the actuation rod 5 as well, is increased. As a result, the force exerted on the forceps 4 is increased as well, after which this is confirmed by a measured signal from the Fibre Bragg Grating 16. Then, the processing unit 12 is configured to dictate the actuator 21 to continue the application of the same force to the connection element 15. In case the overall force exerted on the forceps 4 is too high after the application of the force by the actuator 21, the actuator 21 is dictated by the processing unit 12 to apply a lower force to the connection element 15.

## Claims

1. Surgical instrument (1), wherein the instrument (1) comprises:
- a distal part (2), wherein the distal part (2) comprises an elongated shaft (3), at least one working element (4) and an actuation rod (5), wherein the at least one working element (4) is movably mounted on a distal end (7) of the shaft (3) and wherein a first end (51) of the actuation rod (5) is connected to the at least one working element (4),
- a proximal part (9), wherein the proximal part (9) is releasably connected to the distal part (2) and wherein the proximal part (9) comprises an actuator device (11) releasably connected to a second end (52) of the actuation rod (5), wherein the actuator device (11) is configured to move the at least one working element (4) with respect to the shaft (3) by movement of the actuation rod (5),
- a sensor (16) arranged to transmit a signal that is representative for a force exerted on the at least one working element (4),
wherein the sensor (16) is arranged in the actuator device (11) and is configured to measure force and/or strain exerted on the actuator device (11), wherein the force and/or strain exerted on the actuator device (11) is representative for the force exerted on the at least one working element (4),
**characterized in that** the actuator device (11) comprises a connection element (15) releasably connected to the second end (52) of the actuation rod (5), wherein the sensor (16) is arranged on the connection element (15),
wherein the actuator device (11) comprises a trigger (20), wherein the trigger (20) is configured to transfer a movement to the connection element (15) of the actuator device (11).

2. Surgical instrument (1) according to claim 1, wherein the sensor (16) is an optical sensor.

3. Surgical instrument according to claim 2, wherein the optical sensor is a Fibre Bragg Grating.

4. Surgical instrument (1) according to any of the preceding claims, wherein the connection element (15) comprises a thinned section (30), having a substantially smaller cross sectional area than the cross sectional area in the remainder of the connection element (15) and wherein the sensor (16) is arranged at the thinned section (30).

5. Surgical instrument (1) according to any of the preceding claims, wherein the sensor (16) is configured to measure mechanical strain in the connection element (15) of the actuator device (11).

6. Surgical instrument (1) according to claim 5, wherein the mechanical strain in the connection element (15) of the actuator device (11) is representative for the force exerted on the at least one working element (4).

7. Surgical instrument (1) according to any of the preceding claims, wherein the distal part (2) is sensor-free.

8. Surgical instrument (1) according to any of the preceding claims, wherein the surgical instrument (1) comprises a processing unit (12), wherein the processing unit (12) is configured to determine a force exerted on the at least one working element (4) on the basis of the signal of the sensor (16).

9. Surgical instrument (1) according to any of the preceding claims, wherein a position sensor (14) is arranged in the proximal part (9) and wherein the position sensor (14) is configured to transmit a signal that is representative for the position of the at least one working element (4) with respect to the elongated shaft (3) of the distal part (2).

10. Surgical instrument (1) according to claim 9, wherein the surgical instrument (1) comprises a housing (10) and wherein the position sensor (14) is configured to measure the position of the actuator device (11) with respect to the housing (10).

11. Surgical instrument (1) according to claim 8 and claim 9 or 10, wherein the processing unit (12) is configured to relate the force exerted on the at least one working element (4) to the position of the at least one working element (4) with respect to the elongated shaft (3) of the distal part (2).

12. Surgical instrument (1) according to any of the preceding claims, wherein the actuator device (11) comprises an actuator (21), wherein the actuator (21) is configured to move the connection element (15) of the actuator device (11).

13. Surgical instrument (1) according to claim 12, wherein the processing unit (12) is configured to control the actuator (21), dependent on the value of the force exerted on the at least one working element (4) and the value for the position of the at least one working element (4) with respect to the elongated shaft (3) of the distal part (2).

14. Surgical instrument (1) according to any of the preceding claims, wherein the at least one working element (4) is a forceps.

## Patentansprüche

1. Chirurgisches Instrument (1), wobei das Instrument (1) umfasst:
- einen distalen Teil (2), wobei der distale Teil (2) einen langgestreckten Schaft (3), wenigstens ein Arbeitselement (4) und eine Betätigungsstange (5) umfasst, wobei das wenigstens eine Arbeitselement (4) beweglich an einem distalen Ende (7) des Schafts (3) gelagert ist und wobei ein erstes Ende (51) der Betätigungsstange (5) mit dem wenigstens einen Arbeitselement (4) verbunden ist,
- einen proximalen Teil (9), wobei der proximale Teil (9) lösbar mit dem distalen Teil (2) verbunden ist und wobei der proximale Teil (9) eine Betätigungsvorrichtung (11) umfasst, die lösbar mit einem zweiten Ende (52) der Betätigungsstange (5) verbunden ist, wobei die Betätigungsvorrichtung (11) dafür gestaltet ist, durch Bewegung der Betätigungsstange (5) das wenigstens eine Arbeitselement (4) bezogen auf den Schaft (3) zu bewegen,
- einen Sensor (16), der dafür gestaltet ist, ein Signal, das für eine auf das wenigstens eine Arbeitselement (4) ausgeübte Kraft repräsentativ ist, zu übertragen,
wobei der Sensor (16) in der Betätigungsvorrichtung (11) angeordnet ist und dafür gestaltet ist, auf die Betätigungsvorrichtung (11) ausgeübte Kraft und/oder Belasstung zu messen, wobei die auf die Betätigungsvorrichtung (11) ausgeübte Kraft und/oder Belastung für die auf das wenigstens eine Arbeitselement (4) ausgeübte Kraft repräsentativ ist,
**dadurch gekennzeichnet, dass** die Betätigungsvorrichtung (11) ein Verbindungselement (15) umfasst, das lösbar mit dem zweiten Ende (52) der Betätigungsstange (5) verbunden ist, wobei der Sensor (16) an dem Verbindungselement (15) angeordnet ist,
wobei die Betätigungsvorrichtung (11) einen Auslöser (20) umfasst, wobei der Auslöser (20) dafür gestaltet ist, eine Bewegung auf das Verbindungselement (15) der Betätigungsvorrichtung (11) zu übertragen.

2. Chirurgisches Instrument (1) nach Anspruch 1, wobei der Sensor (16) ein optischer Sensor ist.

3. Chirurgisches Instrument nach Anspruch 2, wobei der optische Sensor ein Faser-Bragg-Gitter ist.

4. Chirurgisches Instrument (1) nach einem der vorstehenden Ansprüche, wobei das Verbindungselement (15) einen verdünnten Abschnitt (30) umfasst, der eine wesentlich kleinere Querschnittsfläche als die Querschnittsfläche in dem restlichen Verbindungselement (15) aufweist, und wobei der Sensor (16) an dem verdünnten Abschnitt (30) angeordnet ist.

5. Chirurgisches Instrument (1) nach einem der vorstehenden Ansprüche, wobei der Sensor (16) dafür gestaltet ist, eine mechanische Belastung in dem Verbindungselement (15) der Betätigungsvorrichtung (11) zu messen.

6. Chirurgisches Instrument (1) nach Anspruch 5, wobei die mechanische Belastung in dem Verbindungselement (15) der Betätigungsvorrichtung (11) für die auf das wenigstens eine Arbeitselement (4) ausgeübte Kraft repräsentativ ist.

7. Chirurgisches Instrument (1) nach einem der vorstehenden Ansprüche, wobei der distale Teil (2) sensorfrei ist.

8. Chirurgisches Instrument (1) nach einem der vorstehenden Ansprüche, wobei das chirurgische Instrument (1) eine Prozessoreinheit (12) umfasst, wobei die Prozessoreinheit (12) dafür gestaltet ist, eine auf das wenigstens eine Arbeitselement (4) ausgeübte Kraft auf Grundlage des Signals des Sensors (16) zu bestimmen.

9. Chirurgisches Instrument (1) nach einem der vorstehenden Ansprüche, wobei ein Positionssensor (14) in dem proximalen Teil (9) angeordnet ist und wobei der Positionssensor (14) dafür gestaltet ist, ein Signal zu übertragen, das für die Position des wenigstens einen Arbeitselements (4) bezogen auf den langgestreckten Schaft (3) des distalen Teils (2) repräsentativ ist.

10. Chirurgisches Instrument (1) nach Anspruch 9, wobei das chirurgische Instrument (1) ein Gehäuse (10) umfasst und wobei der Positionssensor (14) dafür gestaltet ist, die Position der Betätigungsvorrichtung (11) bezogen auf das Gehäuse (10) zu messen.

11. Chirurgisches Instrument (1) nach Anspruch 8 und Anspruch 9 oder 10, wobei die Prozessoreinheit (12) dafür gestaltet ist, die auf das wenigstens eine Arbeitselement (4) ausgeübte Kraft mit der Position des wenigstens einen Arbeitselements (4) bezogen auf den langgestreckten Schaft (3) des distalen Teils (2) in Beziehung zu setzen.

12. Chirurgisches Instrument (1) nach einem der vorstehenden Ansprüche, wobei die Betätigungsvorrichtung (11) ein Betätigungselement (21) umfasst, wobei das Betätigungselement (21) dafür gestaltet ist, das Verbindungselement (15) der Betätigungsvorrichtung (11) zu bewegen.

13. Chirurgisches Instrument (1) nach Anspruch 12, wobei die Prozessoreinheit (12) dafür gestaltet ist, das Betätigungselement (21) abhängig von dem Wert der auf das wenigstens eine Arbeitselement (4) ausgeübten Kraft und dem Wert für die Position des wenigstens einen Arbeitselements (4) bezogen auf den langgestreckten Schaft (3) des distalen Teils (2) zu steuern.

14. Chirurgisches Instrument (1) nach einem der vorstehenden Ansprüche, wobei das wenigstens eine Arbeitselement (4) eine Zange ist.

## Revendications

1. Instrument chirurgical (1), l'instrument (1) comprenant :
- une partie distale (2), la partie distale (2) comprenant un arbre allongé (3), au moins un élément de travail (4) et une tige d'actionnement (5), l'au moins un élément de travail (4) étant monté mobile sur une extrémité distale (7) de l'arbre (3) et une première extrémité (51) de la tige d'actionnement (5) étant reliée à l'au moins un élément de travail (4),
- une partie proximale (9), la partie proximale (9) étant reliée de manière libérable à la partie distale (2) et la partie proximale (9) comprenant un dispositif d'actionnement (11) relié de manière libérable à une deuxième extrémité (52) de la tige d'actionnement (5), le dispositif d'actionnement (11) étant configuré pour déplacer l'au moins un élément de travail (4) par rapport à l'arbre (3) par le mouvement de la tige d'actionnement (5),
- un capteur (16) agencé pour transmettre un signal qui est représentatif d'une force exercée sur l'au moins un élément de travail (4),
le capteur (16) étant agencé dans le dispositif d'actionnement (11) et étant configuré pour mesurer la force et/ou la déformation exercée sur le dispositif d'actionnement (11), la force et/ou la déformation exercée sur le dispositif d'actionnement (11) étant représentative de la force exercée sur l'au moins un élément de travail (4),
**caractérisé en ce que** le dispositif d'actionnement (11) comprend un élément de connexion (15) relié de manière libérable à la deuxième extrémité (52) de la tige d'actionnement (5), le capteur (16) étant agencé sur l'élément de connexion (15),
le dispositif d'actionnement (11) comprenant un déclencheur (20), le déclencheur (20) étant configuré pour transférer un mouvement à l'élément de connexion (15) du dispositif d'actionnement (11).

2. Instrument chirurgical (1) selon la revendication 1, le capteur (16) étant un capteur optique.

3. Instrument chirurgical selon la revendication 2, le capteur optique étant un réseau de Bragg à fibres optiques.

4. Instrument chirurgical (1) selon l'une quelconque des revendications précédentes, l'élément de connexion (15) comprenant une section amincie (30), ayant une surface de section transversale sensiblement plus petite que la surface de section transversale dans le reste de l'élément de connexion (15) et le capteur (16) étant agencé au niveau de la section amincie (30).

5. Instrument chirurgical (1) selon l'une quelconque des revendications précédentes, le capteur (16) étant configuré pour mesurer la déformation mécanique dans l'élément de connexion (15) du dispositif d'actionnement (11).

6. Instrument chirurgical (1) selon la revendication 5, la déformation mécanique dans l'élément de connexion (15) du dispositif d'actionnement (11) étant représentative de la force exercée sur l'au moins un élément de travail (4).

7. Instrument chirurgical (1) selon l'une quelconque des revendications précédentes, la partie distale (2) étant dépourvue de capteur.

8. Instrument chirurgical (1) selon l'une quelconque des revendications précédentes, l'instrument chirurgical (1) comprenant une unité de traitement (12), l'unité de traitement (12) étant configurée pour déterminer une force exercée sur l'au moins un élément de travail (4) sur la base du signal du capteur (16).

9. Instrument chirurgical (1) selon l'une quelconque des revendications précédentes, un capteur de position (14) étant agencé dans la partie proximale (9) et le capteur de position (14) étant configuré pour transmettre un signal qui est représentatif de la position de l'au moins un élément de travail (4) par rapport à l'arbre allongé (3) de la partie distale (2).

10. Instrument chirurgical (1) selon la revendication 9, l'instrument chirurgical (1) comprenant un boîtier (10) et le capteur de position (14) étant configuré pour mesurer la position du dispositif d'actionnement (11) par rapport au boîtier (10).

11. Instrument chirurgical (1) selon la revendication 8 et la revendication 9 ou 10, l'unité de traitement (12) étant configurée pour relier la force exercée sur l'au moins un élément de travail (4) à la position de l'au moins un élément de travail (4) par rapport à l'arbre allongé (3) de la partie distale (2).

12. Instrument chirurgical (1) selon l'une quelconque des revendications précédentes, le dispositif d'actionnement (11) comprenant un actionneur (21), l'actionneur (21) étant configuré pour déplacer l'élément de connexion (15) du dispositif d'actionnement (11).

13. Instrument chirurgical (1) selon la revendication 12, l'unité de traitement (12) étant configurée pour commander l'actionneur (21), en fonction de la valeur de la force exercée sur l'au moins un élément de travail (4) et de la valeur de la position de l'au moins un élément de travail (4) par rapport à l'arbre allongé (3) de la partie distale (2).

14. Instrument chirurgical (1) selon l'une quelconque des revendications précédentes, l'au moins un élément de travail (4) étant une pince.
